# NEUE EUROPÄISCHE PATENTSCHRIFT

(11) **EP 2 673 015 B2**
(45) Veröffentlichungstag und Bekanntmachung des Hinweises auf die Entscheidung über den Einspruch: **28.03.2018**
(45) Hinweis auf die Patenterteilung: 21.01.2015
(21) Anmeldenummer: 12703099.7
(22) Anmeldetag: 07.02.2012
(51) Int. Cl.: A61M 1/00

(54) **VORRICHTUNG ZUR BEREITSTELLUNG VON UNTERDRUCK FÜR MEDIZINISCHE ANWENDUNGEN**
DEVICE FOR PROVIDING VACUUM FOR MEDICAL APPLICATIONS
DISPOSITIF D'OBTENTION D'UN VIDE POUR APPLICATIONS MÉDICALES

(30) Priorität: 10.02.2011 DE 102011011831
(43) Veröffentlichungstag der Anmeldung: 18.12.2013
(73) Patentinhaber: Paul Hartmann AG, 89522 Heidenheim (DE)
(72) Erfinder: CROIZAT, Pierre, 89542 Herbrechtingen (DE); ECKSTEIN, Axel, 89522 Heidenheim (DE); ROETTGER, Marc, 73087 Bad Boll (DE)
(74) Vertreter: DREISS Patentanwälte PartG mbB
(86) Internationale Anmeldenummer: PCT/EP2012/052018
(87) Internationale Veröffentlichungsnummer: WO 2012/107430

(56) Entgegenhaltungen:
- EP-A2- 0 100 672
- WO-A1-2009/103031
- WO-A1-2011/018132
- WO-A1-2011/118888
- US-A1- 2011 168 857

## Beschreibung

Die Erfindung betrifft Vorrichtung zur Bereitstellung von Unterdruck für medizinische Anwendungen, insbesondere zur Unterdruckbehandlung von Wunden am menschlichen oder tierischen Körper, mit einer Unterdruck erzeugenden Einrichtung und einem nach Gebrauch typischerweise wegwerfbaren Behälter zur Aufnahme von Körperflüssigkeiten, insbesondere von aus einer Wunde abgesaugten Wundsekreten, wobei der Behälter lösbar an der Vorrichtung befestigbar ist und wobei ein Anschluss für eine zum Körper führende Saugleitung vorgesehen ist, so dass eine Unterdruckkommunikation zwischen der Unterdruck erzeugenden Einrichtung, dem Behälter und der zum Körper führenden Saugleitung herstellbar ist, und mit einer Halteeinrichtung zum lösbaren Befestigen der Vorrichtung an einer horizontalen oder vertikalen oder schräg geneigten Strebe oder Stange.

Vorrichtungen zur Unterdruckwundbehandlung sind bereits mehrfach beschrieben worden, insbesondere durch US 2004/0073151 A1, WO 2009/047524 A2, WO 2007/030599 A2 oder EP 1 905 465 A1 und durch die nicht vorveröffentlichten Patentanmeldungen DE 10 2009 038 130.9 und DE 10 2009 038 131.7 der Anmelderin. Auch EP 0 100 672 A2 zeigt eine gattungsgemäße Vorrichtung zur Bereitstellung von Unterdruck. Sie umfasst eine Halteeinrichtung in Form einer Befestigungsklammer mit nach unten offenen halbkreisförmigen Haken zum Einhängen an einer horizontalen Strebe. Die Befestigungsklammer ist mit Schrauben an das Gehäuse der Vorrichtung fixiert.

Bei derartigen Vorrichtungen zur Unterdruckbehandlung von Wunden kommuniziert eine Unterdruck erzeugende Einrichtung über eine Saugleitung mit der Wunde oder der Wundumgebung, wobei ein luftundurchlässiges Abdeckmaterial zum luftdichten Verschließen der Wunde und der Wundumgebung vorgesehen ist, so dass ein Unterdruck im Wundraum herstellbar und Flüssigkeiten aus dem Wundraum in den genannten Behälter absaugbar sind.

Der Begriff eines Unterdrucks bezeichnet im Zusammenhang mit der vorliegenden Erfindung einen gegenüber dem Umgebungsluftdruck (atmosphärischer Luftdruck) erniedrigten Luftdruck insbesondere innerhalb eines Wundverbands. Das Abdeckmaterial eines Wundverbands zum luftdichten Verschließen eines Wundraums muss daher so ausgebildet sein, dass es der sich einstellenden Druckdifferenz standzuhalten vermag, damit der Unterdruck im Wundraum überhaupt angelegt und aufrechterhalten werden kann. Im Bereich der Unterdrucktherapie in der Wundbehandlung wird der Unterdruck quantitativ als Druckdifferenz zwischen dem Umgebungsluftdruck und dem unterhalb des Abdeckmaterials angelegten Luftdruck angegeben. Typischerweise beträgt diese Druckdifferenz im Bereich der Unterdrucktherapie höchstens 250 mm Hg (mm Quecksilbersäule) (1 mm Hg = 1 Torr. = 133,322 Pa). Dieser Unterdruckbereich bis höchstens 250 mm Hg hat sich als für die Wundheilung geeignet erwiesen. Ein bevorzugter Unterdruckbereich liegt zwischen 10 und 150 mm Hg.

Der unter Verwendung der Vorrichtung an die Wunde angelegte Unterdruck kann bei einer typischen Unterdruckbehandlung entweder zeitlich im Wesentlichen konstant gehalten werden, oder er kann zeitlich verändert, insbesondere zyklisch verändert werden, was über eine entsprechend ausgebildete und programmierte Steuervorrichtung bei der Unterdruck erzeugenden Einrichtung, insbesondere in Abhängigkeit weiterer Parameter, realisiert werden kann.

Zum Anlegen von Unterdruck und vorzugsweise auch zum Absaugen von Körperflüssigkeiten ist eine vorzugsweise flexible Saugleitung, beispielsweise in Form eines Drainageschlauchs, vorgesehen, der einenends über einen sogenannten Port im Bereich des Wundabdeckmaterials mit der Wundumgebung oder dem Wundraum und anderenends mit dem eingangs erwähnten Behälter zur Aufnahme von Körperflüssigkeiten bzw. mit der Unterdruck erzeugenden Einrichtung kommuniziert.

Neben der Unterdruckwundbehandlung sind auch andere Anwendungen der hier in Rede stehenden Vorrichtung zur Bereitstellung von Unterdruck für medizinische Anwendungen denkbar, insbesondere die Absaugung an sich beliebiger Körperflüssigkeiten, im Bereich der medizinischen Inkontinentenversorgung, der Versorgung von Stoma-Patienten oder im Bereich der Absaugung von Wundsekreten, gegebenenfalls unter Verwendung von Spülflüssigkeiten, auch ohne Anlegen eines Unterdrucks über wesentliche Zeiträume.

Mit WO 2008/036344 A1 wurde bereits der Vorschlag unterbreitet, die Vorrichtung an einer Stange oder Strebe eines Patientenbetts mittels Klemm- oder Knebelschrauben festzuklemmen, wobei ein um 90° verschwenkbarer Klemmarm beschrieben ist, um die Befestigung an einer vertikalen oder horizontalen Strebe des Patientenbetts zu ermöglichen. Auch das simple Anhängen der Vorrichtung über Haken ist vorbekannt.

Der vorliegenden Erfindung liegt die Aufgabe zugrunde, eine betriebssichere und einfach zu realisierende Fixierbarkeit der Vorrichtung zu schaffen.

Diese Aufgabe wird bei einer Vorrichtung der genannten Art erfindungsgemäß durch eine Vorrichtung mit den Merkmalen des Anspruchs 1 gelöst.

Dabei ist die Halteeinrichtung ein an einem Gehäuse der Vorrichtung befestigbares und hiervon lösbares im wesentlichen starres Halteteil und ein biegsames, vorzugsweise elastisch nachgiebiges bandförmiges Haltemittel, wobei das starre Halteteil seinerseits eine Aufnahme zur lösbaren Anordnung des biegsamen bandförmigen Haltemittels aufweist, und das biegsame bandförmige Haltemittel um eine horizontale oder vertikale oder schräg geneigte Strebe oder Stange herumlegbar und auf sich oder auf das Halteteil zurückführbar und schließbar ist, derart dass unter Ausübung von Zugkraft auf das Haltemittel und durch Schließen des Haltemittels eine im wesentlichen spielfreie und dabei klemmschlüssige Fixierung und Vorrichtung gegenüber der Strebe oder Stange erzielbar ist.

Es wird also erfindungsgemäß der Vorschlag unterbreitet, die Halteeinrichtung nicht an der Vorrichtung oder an dem Vorrichtungsgehäuse als unlösbaren Bestandteil zu integrieren, was das gesamte Gewicht der Vorrichtung vergrößern würde. Dies wäre wiederum bei solchen Vorrichtungen zur Bereitstellung von Unterdruck nachteilig, die nicht nur als stationäres Aggregat, sondern auch als am Körper des Patienten tragbare, also mitführbare Vorrichtungen ausgebildet sind. Dadurch, dass die Halteeinrichtung, d. h. das im Wesentliche starre Halteteil, lösbar an einem Gehäuse der Vorrichtung befestigbar ist, kann es mitsamt des biegsamen bandförmigen Haltemittels von dem Gehäuse abgenommen werden, wenn die Vorrichtung im mobilen Betrieb benutzt und von einem Patienten mitgeführt wird.

Zum Befestigen der Halteeinrichtung über deren im Wesentlichen starres Halteteil am Gehäuse der Vorrichtung kommen an sich beliebige formschlüssig und/oder klemmschlüssig wirkenden Arten der Befestigung in Frage, wobei einer Schraubenverbindung der Vorzug gegeben wird. Solchenfalls lässt sich das starre Halteteil mit einer Schraubendurchgangsöffnung ausbilden, so dass das Halteteil über eine vorzugsweise manuell und werkzeuglos festziehbare und lösbare Klemmschraube über eine entsprechend ausgebildete Gewindeöffnung an dem Vorrichtungsgehäuse befestigt werden kann. Das starre Halteteil ist dabei vorteilhafterweise gegen eine Unterseite des Vorrichtungsgehäuses montiert.

Obschon das im Wesentlichen starre Halteteil aus verschiedenen denkbaren Materialien gefertigt werden kann und verschiedene an die Vorrichtung angepasste Geometrien oder Ausgestaltungen haben kann, erweist es sich als vorteilhaft, wenn es aus einem ebenen Flachmaterialabschnitt ausgebildet ist. Es kann sich solchenfalls insbesondere um ein metallisches Biegestanzteil handeln, welches in sehr einfacher Weise und kostengünstig herstellbar ist und zudem eine der Anforderung entsprechende Tragfähigkeit für die Vorrichtung zur Bereitstellung von Unterdruck aufweist.

Wie bereits erwähnt, dient das Halteteil einerseits der Stützung der Vorrichtung und andererseits der Halterung oder Anbringung des biegsamen, vorzugsweise elastisch nachgiebigen bandförmigen Haltemittels, mit dem die Vorrichtung an einer horizontalen oder vertikalen Strebe eines Patientenbetts oder eines Infusionsständers auf einfache Weise befestigbar ist. Hierfür erweist es sich als vorteilhaft, dass in weiterer Ausbildung der Erfindung die an dem Halteteil ausgebildete Aufnahme zur lösbaren Anordnung des biegsamen bandförmigen Haltemittels eine Schiebesitzanordnung bildet, derart, dass das bandförmige Haltemittel in einer linearen Richtung auf das starre Halteteil aufschiebbar ist.

Hierfür erweist es sich weiter als vorteilhaft, dass die an dem Halteteil ausgebildete Aufnahme wenigstens einen fingerförmigen oder zungenförmigen Steg, insbesondere Flachsteg, aufweist, der beim Aufschieben des biegsamen bandförmigen Haltemittels in eine kanalbildende Aussparung des Haltemittels eingreift. Wenn der fingerförmige oder zungenförmige Steg verjüngt, also insbesondere angefast ausläuft, so ist das Aufschieben des biegsamen bandförmigen Haltemittels erleichtert.

Dabei ist die an dem Halteteil ausgebildete Aufnahme so ausgebildet, dass das biegsame bandförmige Haltemittel in zwei 90° zueinander einschließenden Orientierungen einer Bandlängsachse befestigbar ist. Auf diese Weise kann bei unveränderter Positionierung des starren Halteteils eine Befestigung um eine horizontale bzw. vertikale Strebe adaptiert werden.

Alternativ und von besonderer Bedeutung kann vorgesehen sein, dass das im Wesentlichen starre Halteteil wenigstens zwei Aufnahmen zur lösbaren Anordnung je eines biegsamen bandförmigen Haltemittels aufweist, wobei die Aufnahmen so orientiert sind, dass die bandförmigen Haltemittel in einem Winkel von vorzugsweise 90° zueinander und zu der Vorrichtung ausgerichtet sind. Auf diese Weise wird erfindungsgemäß erreicht, dass nicht nur ein, sondern zwei bandförmige Haltemittel in verschiedenen Orientierungen an dem einen starren Halteteil befestigbar sind. Somit stehen insgesamt wenigstens zwei bandförmige Haltemittel zur Fixierung des Vorrichtungsgehäuses an einer Stange oder Strebe zur Verfügung.

Nach einem weiteren Erfindungsgedanken von besonderer Bedeutung wird vorgeschlagen, dass das im Wesentlichen starre Halteteil eine Winkelform aufweist. Solchenfalls erweist es sich in Weiterbildung dieses Gedankens als vorteilhaft, wenn an jedem Schenkel des winkelförmigen Halteteils eine Aufnahme zur lösbaren Anordnung je eines biegsamen, bandförmigen Haltemittels ausgebildet ist. Da diese Aufnahme wie vorausgehend bereits erwähnt in vorteilhafter Weise eine Schiebesitzanordnung bildet, so erweist es sich als vorteilhaft, wenn die Schiebesitzaufnahme bzw. deren fingerförmige oder zungenförmige Stege aufeinander zu in Richtung des Scheitels des Winkels gerichtet sind. Über die Winkelform, die vorzugsweise 90° betragen wird, kann dann eine zusätzliche relative Orientierung der jeweiligen Bandebene des biegsamen bandförmigen Haltemittels im Raum vorgegeben werden, was sich im Hinblick auf eine größtmögliche Adaptierbarkeit der Halteeinrichtung an verschiedene Anbausituationen im praktischen Betrieb als vorteilhaft erweisen kann.

Das biegsame bandförmige Haltemittel ist, wie bereits erwähnt, vorzugsweise elastisch nachgiebig, insbesondere gummielastisch nachgiebig ausgebildet. Es kann daher vorteilhafterweise aus einem gummiartigen, insbesondere silikonartigen Material in vorteilhafter Weise beschaffen sein, wobei auch andere Materialien, insbesondere auf Polymerbasis, oder Verbundmaterialien in Frage kommen.

Um die Anbringbarkeit des biegsamen bandförmigen Haltemittels in vorzugsweise verschiedenen Orientierungen an der Aufnahme des starren Halteteils zweckmäßig und bedienerfreundlich auszubilden, wird vorgeschlagen, dass das biegsame bandförmige Haltemittel eine kanalbildende Aussparung oder zwei vorzugsweise rechtwinklig zueinander orientierte kanalbildende Aussparungen aufweist. Auf diese Weise lässt sich das Haltemittel durch einen einzigen sich jedermann sofort erschließenden Montagevorgang anordnen. Die Vorrichtung ist solchenfalls auch durch den weniger ausgebildeten Patienten ohne weiteres fixierbar oder lösbar, wenn sie etwa im mobilen Betrieb am Körper des Patienten mitgeführt werden soll.

Weiter erweist es sich als vorteilhaft, dass das biegsame bandförmige Haltemittel ein mechanisch wirkendes Verschlussmittel aufweist, so dass es durch Zurückführen auf sich selbst oder auf das Halteteil schließbar ist. Beispielsweise wäre es zumindest grundsätzlich denkbar, dass mechanische Verschlussmittel auf Basis eines Haken/Schlaufenverschlussmaterials zum Einsatz kommen. Im Hinblick auf eine sichere Fixierung erweist es sich indessen als vorteilhaft, wenn das mechanisch wirkende Verschlussmittel von wenigstens einem Vorsprung einerseits und von Durchgangsöffnungen andererseits gebildet ist, in die der Vorsprung dann eingreifen kann, um eine unter Vorspannung stehende sichere formschlüssige Verschlusswirkung zu erzielen. Die vorzugsweise mehreren Durchgangsöffnungen sind dann vorzugsweise entsprechend einem Raster in dem Bandmaterial des bandförmigen Haltemittels vorgesehen und ausgebildet. Der genannte wenigstens eine Vorsprung kann dabei an dem starren Halteteil und/oder an dem biegsamen bandförmigen Haltemittel ausgebildet sein. Nach einer Ausführungsform erstreckt sich der wenigstens eine Vorsprung in einer Ebene des biegsamen bandförmigen Haltemittels oder in einer Ebene des starren Halteteils.

Nach einem weiteren besonders vorteilhaften Erfindungsgedanken wird vorgeschlagen, dass das biegsame bandförmige Haltemittel ein starres, vorzugsweise metallisches, insbesondere als eingespritztes Einlegeteil ausgebildetes Verstärkungsteil aufweist. Dieses Verstärkungsteil ist vorzugsweise flächenhaft und mit Öffnungen zum besseren Umspritzen oder Einlaminieren ausgebildet. Solchenfalls erweist es sich als vorteilhaft, wenn das Verstärkungsteil den wenigstens einen Vorsprung bildet, der Vorsprung also einstückig mit diesem Verstärkungsteil ausgebildet ist. Solchenfalls erstreckt sich der Vorsprung über eine Oberfläche oder einen Rand des das bandförmige Haltemittel bildenden Materials hinaus.

Weiter erweist es sich als besonders vorteilhaft, wenn das biegsame bandförmige Haltemittel einen verdickten Bereich aufweist, der quer zur Bandebene 3-dimensional konturiert ausgebildet ist, um eine nachgiebige Anlagezone zur Anlage an eine Stange oder Strebe zu bilden. Solchenfalls ist die Anlagezone zweckmäßigerweise auf der dem Halteteil gegenüberliegenden Seite des biegsamen bandförmigen Haltemittels ausgebildet.

Nach einem weiteren Erfindungsgedanken ist das starre Halteteil so ausgebildet, dass zwischen dessen Aufnahme für das bandförmige Haltemittel und dem Gehäuse der Vorrichtung der lösbare Behälter zur Aufnahme von Flüssigkeit vorgesehen ist. Für diese Ausbildung eignet sich in besonderem Maße die eingangs erwähnte winkelförmige Gestalt des starren Halteteils, wodurch in Tiefenrichtung der Bauraum zur lösbaren Anordnung des Behälters unterfangen oder überfangen werden kann.

Im Übrigen erweist es sich als vorteilhaft, wenn die Vorrichtung zur Bereitstellung von Unterdruck für medizinische Anwendungen so ausgebildet ist, wie dies in den nicht vorveröffentlichten Patentanmeldungen DE 10 2009 038 130.9 und DE 10 2009 038 131.7 der Anmelderin beschrieben ist. Es wird insoweit auf den gesamten Offenbarungsgehalt dieser Anmeldungen hinsichtlich der Ausgestaltung der Vorrichtung mit Ausnahme der dort nicht beschriebenen Halteeinrichtung Bezug genommen. Diese Offenbarung soll durch Bezugnahme zum Gegenstand der vorliegenden Anmeldung gemacht werden. Dort ist insbesondere folgendes beschrieben:
Insbesondere erweist es sich als vorteilhaft, wenn beide Gehäuseteile im wesentlichen scheibenförmig ausgebildet sind und über eine im wesentlichen vertikal orientierte Trennebene gegeneinander anliegen. Die scheibenförmige Ausbildung der beiden Gehäuseteile bedeutet im Sinne der vorliegenden Erfindung, dass die Gehäuseteile, wenn sie in einem stationären Betrieb mit ihrer Standseite bestimmungsgemäß auf einer horizontalen Unterlage abgestellt sind, jeweils eine Breite und Höhe aufweisen, die gegenüber einer Tiefe in horizontaler Richtung betrachtet größer sind. Die Gehäuseteile liegen also, wenn sie lösbar gegeneinander fixiert sind, mit ihren in der Scheibenebene liegenden Seiten gegeneinander an. Wenn vorausgehend von einer Trennebene die Rede ist, so bedeutet dies aber nicht zwangsläufig, dass die Gehäuseteile über eine exakte ebene Fläche gegeneinander anliegen. Vielmehr können in vorteilhafter Weise als Zentriermittel wirkende Vorsprünge oder dergleichen Formgebung in vorzugsweise komplementärer Form zueinander bei den Gehäuseteilen vorgesehen sein. Erfindungsgemäß werden die beiden Gehäuseteile also nicht ineinander gestülpt oder übereinander gestapelt, sondern sie liegen über die genannte im Wesentlichen vertikal orientierte Trennebene nebeneinander und gegeneinander an, wenn die Vorrichtung bestimmungsgemäß mit ihrer Standseite auf einer horizontalen Oberfläche abgestellt ist oder am Körper eines aufrecht stehenden Menschen getragen wird. Im letzteren Fall ist der erste Gehäuseteil im am Körper des Benutzers mitgeführten Zustand der Vorrichtung (mobiler Betrieb) körperabgewandt und der zweite Gehäuseteil körperzugewandt positioniert.

Dadurch dass der den zweiten Gehäuseteil bildende oder in oder an dem zweiten Gehäuseteil angeordnete Behälter zusammen mit dem zweiten Gehäuseteil im mobilen Betrieb der Vorrichtung körperzugewandt und der die Unterdruck erzeugende Einrichtung aufnehmende erste Gehäuseteil körperabgewandt orientiert ist, ist es möglich, den zweiten Gehäuseteil gewissermaßen mittels des ersten Gehäuseteils abzudecken. Auf diese Weise könnte der zweite Gehäuseteil oder der Behälter auch durchsichtig ausgebildet werden, ohne dass eine unmittelbare Einsichtnahme durch dritte Personen möglich wäre. Des Weiteren gewährt die Anordnung des zweiten Gehäuseteils im Bereich des Körpers größere Gestaltungsmöglichkeiten zur Anpassung an den Körper als dies bei dem ersten die Unterdruck erzeugende Einrichtung aufnehmenden Gehäuseteil der Fall wäre. Weiter kann durch die körperabgewandte Anordnung des ersten Gehäuseteils der Zugang zu Bedienelementen für die Unterdruck erzeugende Einrichtung und deren Steuerung im Bereich der Sichtseite der Vorrichtung vorgesehen werden. Hierdurch ist auch der Zugriff durch den Benutzer der Vorrichtung selbst möglich, indem die Bedienkomponenten und möglicherweise auch Anzeigekomponenten dann körperabgewandt und vorzugsweise von oben einsehbar angeordnet sind.

Im Hinblick auf ein benutzerfreundliches Fügen des ersten und des zweiten Gehäuseteils erweist es sich als vorteilhaft, wenn der erste und der zweite Gehäuseteil an ihren einander zugewandten Seiten Zentriermittel aufweisen und die Gehäuseteile im Wesentlichen quer zu der Trennebene gegeneinander fügbar sind. Die genannten Zentriermittel können in an sich beliebiger Weise etwa durch stift- oder konusförmige Elemente oder durch vorstehende und zurückgesetzte Gehäusebereiche gebildet sein, die insbesondere auch block- oder kastenförmig ausgebildet sein können und vorzugsweise komplementär und mit entsprechenden Anlaufschrägen versehen miteinander in einem zentrierenden Sinn zusammenwirken. Vorzugsweise sind diese Zentriermittel bzw. die gegeneinander anliegenden Seiten der Gehäuseteile so ausgebildet, dass sie sich nur in der korrekten Weise fügen lassen.

Zum Fügen der Gehäuseteile erweist es sich als vorteilhaft, wenn der eine Gehäuseteil von oben leicht schräg zur Vertikalen auf ein Auflager des anderen Gehäuseteils aufsetzbar und dann im wesentlichen quer zu der Trennebene in Anlage gegen den anderen Gehäuseteil schwenkbar ist.

Es erweist sich auch als vorteilhaft, wenn beim Fügen des ersten und zweiten Gehäuseteils zugleich eine Unterdruckkommunikation zwischen Behälter und Unterdruck erzeugender Einrichtung hergestellt wird, so dass der Fügevorgang insgesamt benutzerfreundlich wird und der Benutzer nicht in Kontakt mit Körperflüssigkeiten gelangt. In entsprechender Weise kommt diesem Gedanken beim Lösen der Gehäuseteile nach Gebrauch noch größere Bedeutung zu.

Um die beiden Gehäuseteile sicher aneinander zu halten, erweist es sich als vorteilhaft, wenn sie durch schnappende, rastende oder in sonstiger Weise formschlüssig wirkende Verriegelungs- oder Hintergriffsmittel gegeneinander lösbar fixierbar sind.

Nach einer Ausführungsform der Erfindung erweist es sich als vorteilhaft, dass das Verriegelungs- oder Hintergriffsmittel von der Trennebene eines der Gehäuseteile vorsteht und beim Fügen der Gehäuseteile quer zur Fügerichtung der Gehäuseteile auslenkbar und in eine Hintergriffsstellung bringbar ist.

Das Verriegelungs- oder Hintergriffsmittel ist in weiterer Ausbildung der Erfindung durch ein manuell bedienbares Betätigungsorgan in Freigaberichtung bringbar. Das manuell bedienbare Betätigungsorgan kann grundsätzlich an beliebiger Stelle der Vorrichtung angeordnet sein. Es erweist sich jedoch als vorteilhaft, wenn es an einer Oberseite der Vorrichtung vorgesehen und insbesondere als eindrückbarer Taster ausgebildet ist. Auf diese Weise ist es nämlich möglich, dass die Vorrichtung auf einer vorzugsweise horizontalen Unterlage abgestellt wird und dann das Betätigungsorgan erfolgreich betätigt werden kann, ohne dass die Vorrichtung seitlich wegrutscht.

Zum Halten, Anheben oder Handhaben der Vorrichtung als Ganzes und/oder nur des einen oder anderen Gehäuseteils erweist es sich als vorteilhaft, wenn der eine oder andere Gehäuseteil für einen manuellen Eingriff ausgebildet ist, so dass die Vorrichtung oder nur der betreffende Gehäuseteil hierdurch ergriffen und von dem anderen Gehäuseteil gelöst werden kann. Es erweist sich als besonders vorteilhaft, wenn der zweite Gehäuseteil für diese Zwecke eine Eingriffsmulde oder Eingriffsvertiefung aufweist. Diese Eingriffsmulde oder Eingriffsvertiefung ist vorzugsweise im Bereich eines manuell bedienbaren Betätigungsorgans für ein Verriegelungs- oder Hintergriffsmittel vorgesehen, so dass ein Benutzer oder eine Pflegeperson mit nur einer Hand den zweiten Behälterteil entriegeln und vom ersten Behälterteil lösen kann. Diese Eingriffsmulde oder Eingriffsvertiefung ist vorzugsweise in einem oberen und vom Körper weg geneigten Bereich des zweiten Gehäuseteils auf der dem Körper zugewandten Seite des zweiten Gehäuseteils vorgesehen. Auf diese Weise ist es einem Benutzer ohne weiteres möglich, ausgehend vom mobilen Betrieb, die Vorrichtung mit einer Hand zu ergreifen und mit der anderen Hand ein später zu erörterndes insbesondere gurtartiges Befestigungsmittel zu lösen.

Im Hinblick auf eine hohe Standfestigkeit der Vorrichtung im stationären Betrieb oder auch beim zeitweiligen Absetzen der Vorrichtung auf einer vorzugsweise ebenen Fläche erweist es sich als vorteilhaft, wenn ein Schwerpunkt der Vorrichtung (im noch ungebrauchten Zustand des Behälters) in der unteren Hälfte der Vorrichtung, also unterhalb einer gedachten Horizontalmittelebene auf halber Höhe der Vorrichtung liegt.

Im Hinblick darauf erweist es sich auch als vorteilhaft, wenn das Verhältnis einer Standfläche der Vorrichtung zu ihrer Höhe 4,0 - 6,0 cm²/cm, insbesondere 4,5 - 5,5 cm²/cm beträgt. Die Standfläche der Vorrichtung kann beispielsweise ca. 81 cm² und ihre Höhe ca. 16 cm betragen.

Es wäre denkbar und für viele Anwendungen vorteilhaft, wenn die Standfläche der Vorrichtung im aneinander gefügten Zustand ihrer beiden Gehäuseteile von beiden Gehäuseteilen gebildet ist. Nach einer weiteren Ausführungsform der Erfindung hat es sich jedoch als hinreichend erwiesen, wenn die Standfläche der Vorrichtung nur von dem ersten Gehäuseteil gebildet ist. Hierfür erweist es sich als vorteilhaft, wenn das erste Gehäuseteil einen in eine Formausnehmung des ersten Gehäuseteils vorspringenden Bereich aufweist, der einen Teil der Standfläche bildet. Auf diese Weise wird nämlich erreicht, dass die Standfestigkeit des die Unterdruck erzeugende Einrichtung nebst Steuerkomponenten aufnehmenden ersten Gehäuseteils in quasi unveränderter Form auch nach dem Abnehmen des zweiten Gehäuseteils gegeben ist.

Nach einem weiteren Erfindungsgedanken wird vorgeschlagen, die Vorrichtung so auszubilden, dass der erste Gehäuseteil den zweiten Gehäuseteil in frontaler Blickrichtung auf eine körperabgewandte Sichtseite des ersten Gehäuseteils vorzugsweise vollständig oder zu wenigstens 90% der Aufsichtsfläche in dieser Blickrichtung verdeckt. Dies eröffnet wiederum weitere Möglichkeiten, den zweiten Gehäuseteil preiswert auszubilden, da er nur zum geringen Teil im Bereich seiner seitlichen Flächen für den Benutzer oder für Dritte einsehbar ist, wenn die Vorrichtung am Körper des Benutzers getragen wird. Der Behälter muss also nicht mehr in Tragebeuteln "versteckt" werden.

Nach einem Erfindungsgedanken von besonderer Bedeutung wird vorgeschlagen, dass die Vorrichtung gewissermaßen als Sachgesamtheit mehrere zweite Gehäuseteile mit einem jeweiligen Behälter umfasst, die wahlweise zur Bildung der Vorrichtung mit dem ersten Gehäuseteil verbindbar sind, wobei die zweiten Gehäuseteile und deren Behälter unterschiedliche Größe, insbesondere in Tiefenrichtung, also in Richtung auf den Benutzer, aufweisen. Es können auf diese Weise unterschiedliche Aufnahmekapazitäten durch die verschiedenen Gehäuseteile und deren Behälter bereitgestellt werden. Hierbei können Gehäuseteile vorgesehen werden, die sich für einen mobilen Betrieb eignen und solche, die eher für einen stationären Betrieb bestimmt sind, also insbesondere großvolumiger ausgebildet sind, damit die Behälter zur Aufnahme von Flüssigkeit weniger häufig ausgetauscht werden müssen.

Es erweist sich weiter als vorteilhaft, wenn die Halteeinrichtung und ihr starres Halteteil so ausgebildet und an dem Gehäuse fixierbar sind, dass der Behälter oder auch unterschiedlich dimensionierte Behälter, ohne die Halteeinrichtung für die Vorrichtung lösen zu müssen, von dem Gehäuse abgenommen oder daran angesetzt werden können. Für den Fall, dass unterschiedlich dimensionierte Behälter zum Einsatz kommen, ist die Halteeinrichtung und ihr starres Halteteil so ausgebildet, dass die unterschiedlich dimensionierten Behälter gleichermaßen "Platz haben" und ausgetauscht werden können.

Weiter erweist es sich als vorteilhaft, wenn eine Füllstandsanzeigeeinrichtung für den Behälter vorgesehen ist. Diese Füllstandsanzeigeeinrichtung kann beispielsweise durch eine hinreichend durchsichtige Ausbildung einer Gehäusewandung des zweiten Gehäuseteils verwirklicht sein, oder sie kann in sonstiger, an sich beliebiger Weise, insbesondere über elektronische Sensoren und Anzeigemittel gebildet sein. Weiter erweist sich eine Überwachungseinrichtung als vorteilhaft, welche in Abhängigkeit von dem erreichten Füllstand im Behälter eine Alarmmeldung ausgibt. Der Behälter des zweiten Gehäuseteils kann ein superabsorbierendes Medium enthalten, um die angesaugte Flüssigkeit zu binden und ein Schwappen der Flüssigkeit zu verhindern.

Es wurde bereits darauf hingewiesen, dass die erfindungsgemäße Ausbildung der Vorrichtung es in vorteilhafter Weise gestattet, dass an einer körperabgewandten Sichtseite des ersten Gehäuseteils Bedienelemente und Anzeigeelemente für die Unterdruck erzeugende Einrichtung vorgesehen sind. In vorteilhafter Weise sind die Bedienelemente in Form eines Touchscreens ausgebildet.

Nach einem weiteren Erfindungsgedanken von wesentlicher Bedeutung erweist es sich als vorteilhaft, dass im Wesentlichen die gesamte körperabgewandte Sichtseite des ersten Gehäuseteils von einer flächenhaften Abdeckung gebildet oder überfangen ist, so dass schmutzaufnehmende Fugen im Bereich der Bedienelemente vermieden werden.

Weiter erweist es sich als vorteilhaft, wenn die Gehäuseteile im gefügten Zustand der Vorrichtung mit Ausnahme des Anschlusses für die Saugleitung oder einen Mess- oder Spülkanal und einer etwaigen Eingriffsmulde oder Eingriffsvertiefung keine hintergreifbaren Komponenten an der Außenseite aufweisen.

Weitere Merkmale, Einzelheiten und Vorteile ergeben sich aus der zeichnerischen Darstellung und nachfolgenden Beschreibung einer erfindungsgemäßen Vorrichtung zur Bereitstellung von Unterdruck für medizinische Anwendungen mit einer Halteeinrichtung zur lösbaren Befestigung der Vorrichtung an einer Strebe oder Stange oder dergleichen. In der Zeichnung zeigt:
- Figuren 1 a bis e: verschiedene Ansichten einer bevorzugten Ausführungsform einer am Körper tragbaren Vorrichtung zur Bereitstellung von Unterdruck für medizinische Anwendungen;
- Figuren 2a bis e: verschiedene Ansichten eines eine Unterdruck erzeugende Einrichtung und Steuerungskomponenten umfassenden ersten Gehäuseteils der Vorrichtung nach Figur 1;
- Figuren 3a bis i: verschiedene Ansichten eines einen Behälter zur Aufnahme von Körperflüssigkeiten bildenden zweiten Gehäuseteils der Vorrichtung nach Figur 1;
- Figuren 4a bis e: den Figuren 1a bis e entsprechende Ansichten einer weiteren Ausführungsform einer Vorrichtung, wobei der zweite Gehäuseteil größer dimensioniert ist als bei der Vorrichtung nach Figuren 1a bis e; und
- Figur 5: eine Schnittansicht durch die Vorrichtung im Bereich der Unterdruckkommunikation zwischen dem ersten und zweiten Gehäuseteil;
- Figuren 6, 7: eine Ansicht der zweiten Gehäuseteile der Vorrichtungen nach Figuren 1 und 4 von unten zur Verdeutlichung der unterschiedlichen Standflächen dieser zweiten Gehäuseteile;
- Figur 8: zeigt eine erfindungsgemäße Vorrichtung mit einem starren Halteteil einer Halteeinrichtung;
- Figuren 9a, b: zwei Ansichten des starren Halteteils nach Figur 8;
- Figuren 10a-c: Ansichten eines biegsamen bandförmigen Haltemittels der Halteeinrichtung;
- Figuren 11a, b: verdeutlichen die Orientierung des bandförmigen Haltemittels an dem starren Halteteil.

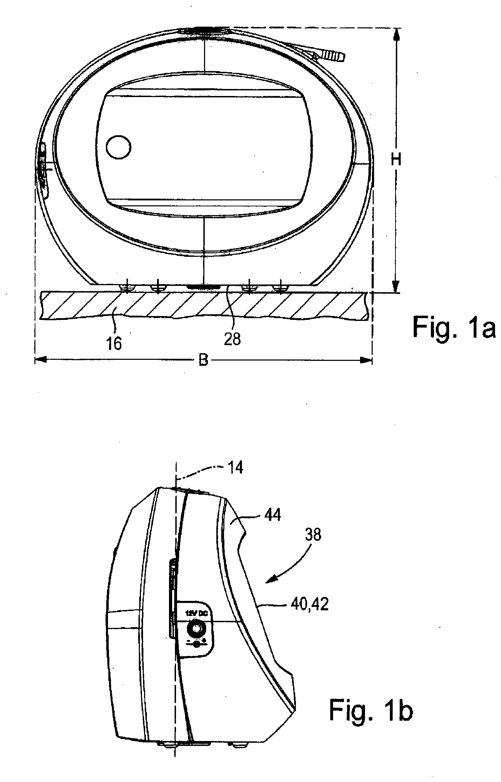

Die Figuren 1a bis e zeigen eine erste Ausführungsform einer tragbaren Vorrichtung 2 zur Bereitstellung von Unterdruck für medizinische Anwendungen. Die Vorrichtung umfasst einen ersten Gehäuseteil 4, in dem eine Unterdruck erzeugende Einrichtung in Form einer Luftpumpe sowie elektrische und elektronische Steuerkomponenten für die Vorrichtung insgesamt aufgenommen sind, einschließlich Batterien oder vorzugsweise wiederaufladbare Akkus. Ein Ladeanschluss für die Akkus ist mit Bezugszeichen 6 bezeichnet. Des Weiteren umfasst die Vorrichtung 2 ein zweites Gehäuseteil 8, welches im bevorzugten Fall zugleich einen Behälter 10 zur Aufnahme von Körperflüssigkeiten, insbesondere zur Aufnahme von aus einer Wunde abgesaugten Wundsekreten, bildet. Bevorzugtermaßen ist der gesamte zweite Gehäuseteil 8 als wegwerfbarer Einmalartikel ausgebildet. An seinem oberen Bereich ist ein Anschlussstutzen 12 für eine nicht dargestellte Saugleitung vorgesehen, die dann beispielsweise bei der Verwendung der Vorrichtung 2 zur Unterdrucktherapie von Wunden zu einem die Wunde druckdicht verschließenden Wundverband führt und dort beispielsweise über einen Port mit dem Wundraum kommuniziert, um im Wundraum einen Unterdruck anzulegen und aufrechtzuerhalten und Wundsekrete in den Behälter 10 abzusaugen. Hierfür kommuniziert der Behälter 10 mit der Unterdruck erzeugenden Einrichtung. Weiter ist ein Anschluss 13 für einen optionalen Mess- oder Spülkanal, der wie die Saugleitung zur Wunde führt, dargestellt. Dieser Anschluss durchgreift den zweiten Gehäuseteil 8 und mündet in den ersten Gehäuseteil 4, von wo aus der Mess- oder Spülkanal beispielsweise mit Luft als Spülmedium beaufschlagt werden kann und/oder ein Druck in diesem Mess- oder Spülkanal detektiert und ausgewertet werden kann.

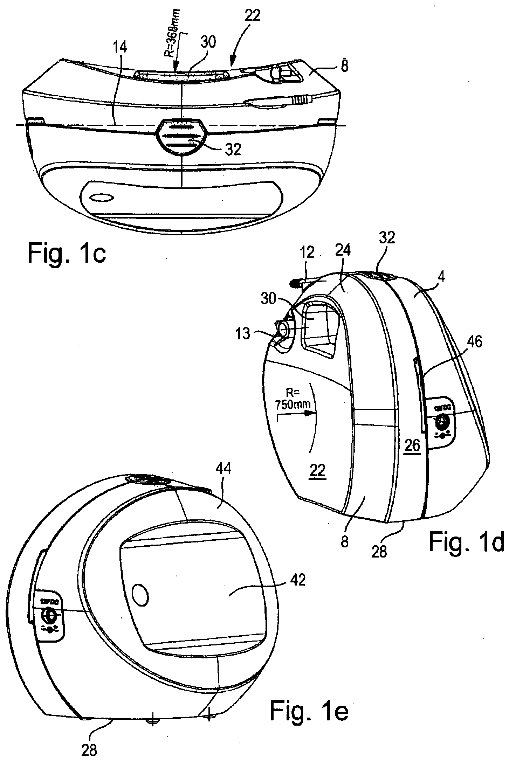

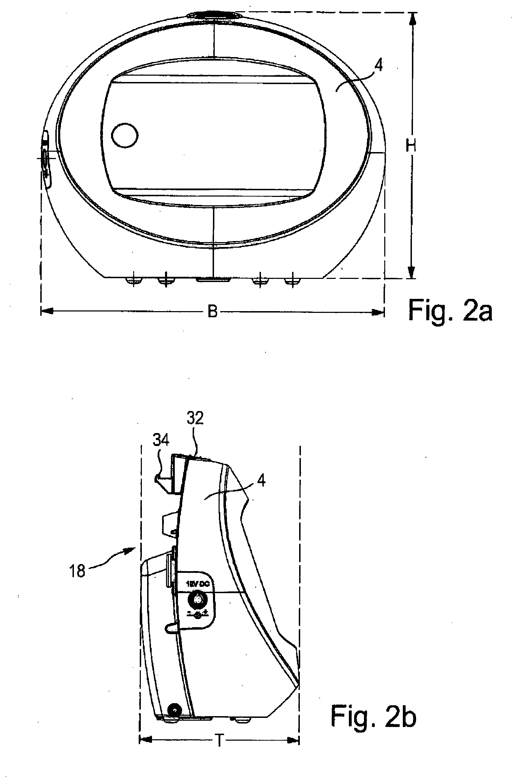

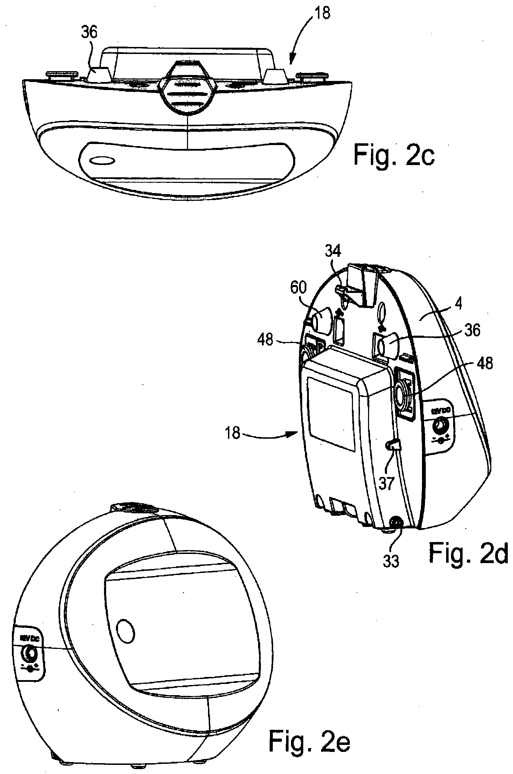

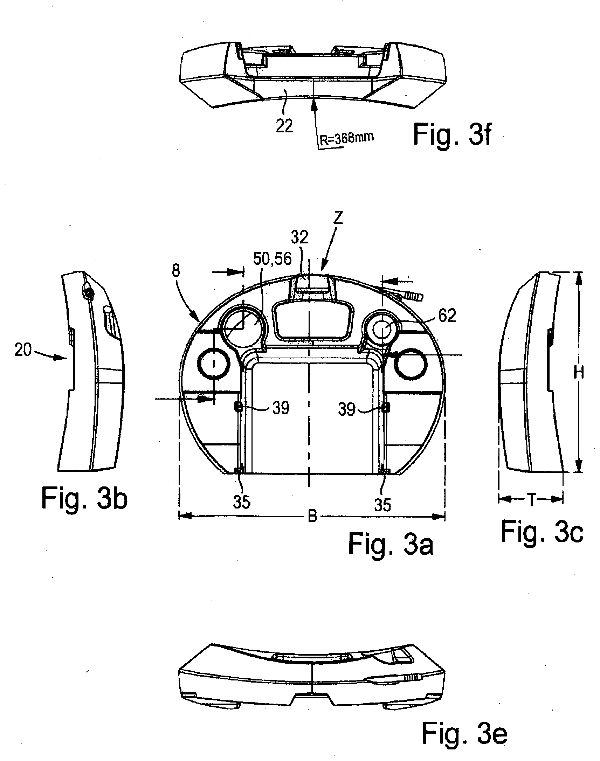

Im bevorzugten dargestellten Fall liegen die Gehäuseteile 4 und 8 über eine im Wesentlichen vertikale Trennebene 14, die in verschiedenen Figuren angedeutet ist, gegeneinander an. Wenn die Vorrichtung 2, wie in Figur 1a angedeutet, auf einer ebenen horizontalen Unterlage 16 abgestellt ist, so ist die Trennebene 14 im Wesentlichen vertikal orientiert. Dies bedeutet, dass die beiden Gehäuseteile 4, 8 nicht ineinander eingesetzt oder übereinander gestapelt sind, sondern dass sie nebeneinander im bestimmungsgemäß zusammengesetzten Zustand der Vorrichtung 2 zu liegen kommen. Der Begriff der Trennebene 14 ist also nicht in dem Sinn zu verstehen, dass es sich um eine geometrisch ebene Fläche handeln muss, was unmittelbar aus den Figuren 2a bis e ersichtlich wird, welche den ersten Gehäuseteil 4 in verschiedenen Ansichten zeigen. Man erkennt sofort, dass die dem zweiten Gehäuseteil 8 zugewandte Seite 18 des ersten Gehäuseteils 4 keinesfalls eben, sondern mit einer Vielzahl von in Richtung auf den zweiten Gehäuseteil 8 vorspringenden Elementen ausgebildet ist. Die dem ersten Gehäuseteil 4 zugewandte Seite 20 des zweiten Gehäuseteils 8 ist im Wesentlichen komplementär zu der Ausbildung der Seite 18 des ersten Gehäuseteils 4 ausgebildet, so dass die beiden Gehäuseteile 4, 8 nur in einer korrekten Weise miteinander gefügt bzw. aneinander befestigt werden können. Die beiden Gehäuseteile 4, 8 sind insgesamt scheibenförmig ausgebildet, d. h. ihre Breite B in horizontaler Richtung und ihre Höhe H in vertikaler Richtung sind jeweils größer als ihre Tiefe T in horizontaler Richtung und senkrecht zur Breitenerstreckung. Hierdurch ist es möglich, dass die Vorrichtung 2 in Tiefenrichtung insgesamt so ausgebildet und bemessen werden kann, dass sie bequem am Körper eines Benutzers getragen werden kann. Erfindungsgemäß ist die Vorrichtung 2 so ausgebildet, dass die nebeneinander angeordneten Behälterteile 4, 8 derart am Körper positionierbar sind, dass der zweite Behälterteil 8 körperzugewandt, also zwischen dem Körper und dem ersten Gehäuseteil 4, zu liegen kommt und der erste Gehäuseteil 4 körperabgewandt zu liegen kommt, also im Wesentlichen die Sichtseite der Vorrichtung 2 bildet. Daher ist die dem Körper des Benutzers zugewandte Seite 22 des zweiten Gehäuseteils 8 verrundet ausgebildet. Wie sich den Figuren 1c, 1d, 3f, 3e entnehmen lässt, ist die körperzugewandte Seite 22 im Schnitt mit einer horizontalen Ebene betrachtet konkav ausgebildet und umfasst im beispielhaft dargestellten Fall abschnittsweise einen Krümmungsradius R von beispielhaft 368 mm (Figur 1c, 3f). Zusätzlich ist die körperzugewandte Seite 22 im Schnitt mit einer vertikalen Ebene betrachtet ebenfalls konkav ausgebildet und weist dort einen Krümmungsradius R von beispielhaft 750 mm auf (Figur 1d). Auf diese Weise lässt sich die Vorrichtung 2 ergonomisch im Hüftbereich eines Benutzers anordnen und tragen.

Man erkennt des Weiteren, dass der zweite Gehäuseteil 8 auf seiner körperzugewandten Seite 22 in einem oberen Bereich und auch seitlich eine Abschrägung 24 weg vom Körper des Benutzers in Richtung auf den ersten Gehäuseteil 4 bzw. in Richtung auf Seitenwandungen 26 oder eine Umfangsstirnseite der Scheibenform des zweiten Gehäuseteils 8 hin umfasst. Die Abschrägung 24 ist im beispielhaft dargestellten Fall umlaufend ausgebildet; sie erstreckt sich ausgehend von der Standseite 28 von unten nach oben, verläuft dort bogenförmig zur anderen Seite und dann wieder nach unten zur Standseite 28 zurück.

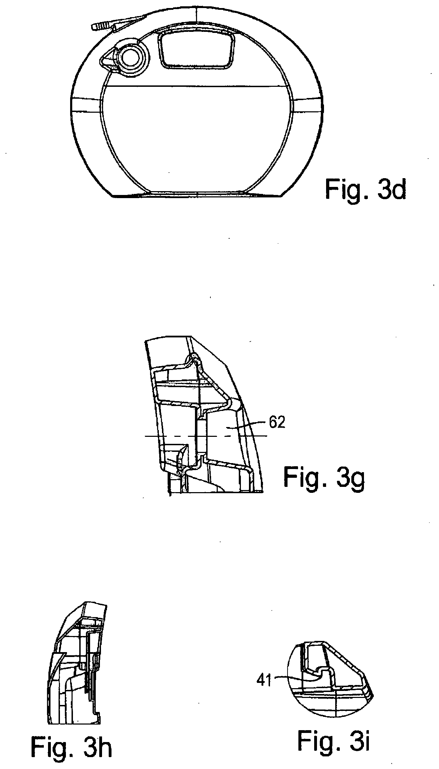

Man erkennt des Weiteren aus den Figuren 1d und 3, dass auf der körperzugewandten Seite 22 des zweiten Gehäuseteils 8 eine Eingriffsvertiefung 30 in Form einer durch den zweiten Gehäuseteil 8 hindurch gehenden Öffnung ausgebildet ist, und zwar in einem oberen leicht vom Körper weg geneigten Bereich des zweiten Gehäuseteils 8. Hierdurch kann die Vorrichtung 2 insgesamt oder nur deren zweiter Gehäuseteil 8 mit einer Hand ergriffen und gehandhabt werden.

Bei der dargestellten bevorzugten Ausführungsform ist nahe bei dieser Eingriffsvertiefung 30 in einer Oberseite der Vorrichtung 2 ein manuell bedienbares Betätigungsorgan 32, beispielsweise in Form eines Tasters, vorgesehen, der auf ein Verriegelungs- oder Hintergriffsmittel 34 einwirkt (s. Figuren 2b und 2d). Im gefügten Zustand der beiden Gehäuseteile 4 und 8 befindet sich das Verriegelungs- oder Hintergriffsmittel 34 in einem die beiden Gehäuseteile 4, 8 formschlüssig aneinander haltenden verriegelten Zustand. Erst durch Betätigen des Betätigungsorgans 32 wird die Verriegelung gelöst, so dass die Gehäuseteile 4, 8 voneinander separiert werden können. Durch die Anordnung und Ausbildung der Eingriffsvertiefung 30 und des manuell bedienbaren Betätigungsorgans 32 in räumlicher Nähe zueinander und derart, dass ein Benutzer sowohl in die Eingriffsvertiefung 30 eingreifen als auch mit einem Finger derselben Hand gleichzeitig das Betätigungsorgan 32 zu bedienen vermag, wird eine Einhandbedienung zum Lösen des zweiten Gehäuseteils 8 vom ersten Gehäuseteil 4 realisiert. Dies erweist sich als besonders vorteilhaft, da solchenfalls ein mit Körperflüssigkeiten befüllter zweiter Gehäuseteil 8 mit nur einer Hand gelöst und in ein Entsorgungsbehältnis gegeben werden kann.

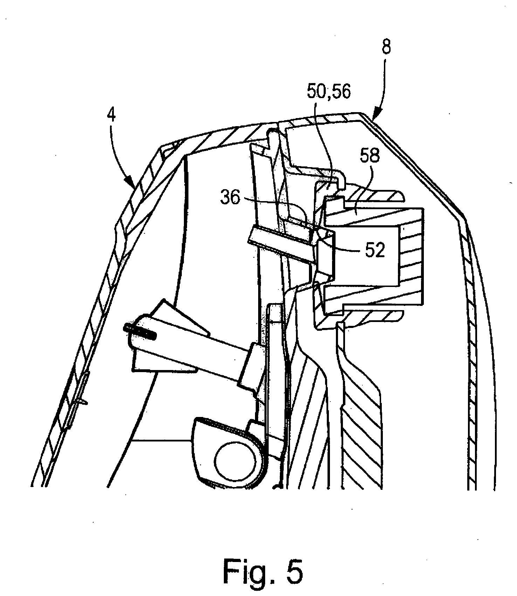

Zum Fügen der beiden Gehäuseteile 4, 8 wird der zweite Gehäuseteil 8 leicht schräg von hinten und von oben mit seinem unteren Rand auf zwei einen Drehpunkt bildende Zapfen 33 (Figur 2d) des ersten Gehäuseteils 4 aufgesetzt. In dem zweiten Gehäuseteil ist hierfür am unteren Rand ein ausgesparter Bereich 35 (Figur 3a) zur Aufnahme des Zapfens 33 ausgebildet. Wenn Zapfen 33 und ausgesparter Bereich 35 miteinander in Eingriff sind, so lässt sich der zweite Gehäuseteil 8 gegen den ersten Gehäuseteil 4 schwenken. Hierdurch werden die einander zugewandten Seiten 18, 20 gegeneinander gelegt und gelangen so selbstzentrierend (unterstützt durch weitere Führungs- oder Zentriermittel 37 (Figur 2d) und 39 (Figur 3a) und die komplementäre Ausbildung der einander zugewandten Seiten 18, 20 der Gehäuseteile 4,8) in ihre bestimmungsgemäße Position. Durch Gegeneinanderbewegen der beiden Gehäuseteile 4, 8, insbesondere im Wesentlichen quer zu der vertikalen Trennebene 14, wird das Verriegelungs- oder Hintergriffsmittel 34 selbsttätig ausgelenkt und rastet dann in seine die Gehäuseteile 4, 8 gegeneinander verriegelnde Stellung. Hierfür ist an dem zweiten Gehäuseteil 8 ein Rasthaken 41 (Figur 3i) vorgesehen, der von dem Verriegelungs- oder Hintergriffsmittel 34 untergriffen wird. Wenn die Gehäuseteile 4, 8 in ihre verriegelte Stellung gebracht werden, wird dann auch automatisch eine Unterdruckkommunikation zwischen dem Inneren des Behälters 10 des zweiten Gehäuseteils 8 und der Unterdruck erzeugenden Einrichtung über Anschlussmittel 36 hergestellt (später im Zusammenhang mit Figur 5 beschrieben).

Eine körperabgewandte Sichtseite 38 des ersten Gehäuseteils 4 ist leicht zur Vertikalen geneigt ausgebildet, so dass sich die Scheibenform nach oben hin verjüngt. Auf diese Weise ist eine leichtere Einsichtnahme auf die Sichtseite 38 gegeben. Dort sind Bedienelemente 40 und Anzeigeelemente 42 insbesondere in Form eines Touchscreens vorgesehen. Im Wesentlichen die gesamte Sichtseite 38 ist von einer flächenhaften Abdeckung 44 überfangen oder gebildet, so dass im Bereich der Bedienelemente 40 keine Schmutz aufnehmenden Fugen gebildet werden.

Des Weiteren zeigen die Figuren im Bereich der Trennebene 14 zwischen den gegeneinander anliegenden Gehäuseteilen 4, 8 einen Einsteckschlitz 46 zum Einstecken und lösbaren Fixieren eines Befestigungsmittels, insbesondere und vorzugsweise in Form eines flexiblen Gürtels, oder eines Bügels oder einer Lasche, an dem/der beispielsweise ein Gürtel oder ein Schultertragriemen befestigt werden kann, oder in sonstiger Form. Es erweist sich als vorteilhaft, dass dieses Befestigungsmittel von den Gehäuseteilen 4, 8 gelöst werden kann und somit nicht stört, wenn die Vorrichtung 2 im stationären Betrieb, also auf einer vorzugsweise ebenen Unterlage 16 stehend, benutzt wird, etwa wenn ein hiermit zu behandelnder Patient in einem Krankenbett ruht. In Figur 2d sind an der Seite 18 des ersten Gehäuseteils 4 Mittel 48 angedeutet, an denen die in den Einsteckschlitz 46 eingesteckten Befestigungsmittel festlegbar oder gehalten sind.

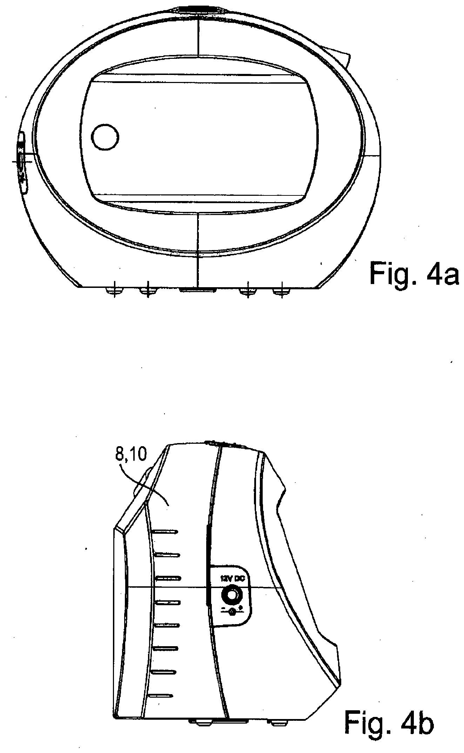

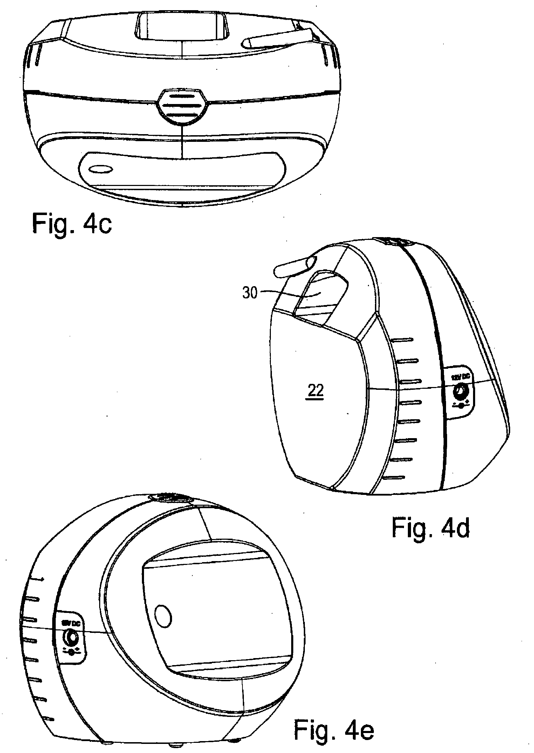

Die in Figuren 4a bis e dargestellte weitere Ausführungsform der erfindungsgemäßen Vorrichtung unterscheidet sich von der in Figuren 3 dargestellten Ausführungsform dadurch, dass der zweite Gehäuseteil 8 und der von ihm gebildete Behälter 10 großvolumiger ausgebildet ist. Die Abschrägung im oberen Bereich der körperzugewandten Seite 22 des zweiten Gehäuseteils 8, wo die Griffmulde 30 ausgebildet ist, ist noch etwas stärker vom Körper des Benutzers weg geneigt. Auf diese Weise ist ein noch besserer Zugriff möglich. Dieser größere zweite Gehäuseteil 8 eignet sich eher für einen stationären Betrieb der Vorrichtung 2; er könnte hierfür auch eine nach außen konvex gewölbte Seite 22 aufweisen oder gar noch ausladender ausgebildet sein als dies die Figuren 4 zeigen.

Figur 5 zeigt im Einzelnen die Ausbildung der Unterdruckkommunikation zwischen dem Inneren des den Behälter 10 bildenden zweiten Gehäuseteils 8 und dem ersten Gehäuseteil 4. Die Ansaugseite einer nicht dargestellten Unterdruck erzeugenden Einrichtung führt zu dem konisch ausgebildeten Anschlussmittel 36, welches sich konisch in Richtung auf den zweiten Gehäuseteil 8 verjüngt. Auf diese Weise kann gegen das konische Anschlussmittel 36 des ersten Gehäuseteils 4 ein wenigstens geringfügig nachgiebig ausgebildetes Gegenanschlussmittel 50 des zweiten Gehäuseteils 8 dichtend angelegt werden, welches im beispielhaft dargestellten Fall eine kreisförmige Öffnung 52 aufweist, die von einer nachgiebigen Dichtlippe 54 begrenzt ist. Dieses Gegenanschlussmittel 50 mündet in das Innere des zweiten Gehäuseteils 8. Es bildet zugleich ein Filteraufnahmemittel 56 für einen Filter 58, der im beispielhaft dargestellten Fall als topfförmiger Filter ausgebildet ist und verhindert, dass Bakterien in den ersten Gehäuseteil 4 eingesogen werden. Man erkennt ohne weiteres, dass beim Gegeneinanderbewegen der beiden Gehäuseteile 4, 8 das Anschlussmittel 36 des ersten Gehäuseteils 4 mit dem Gegenanschlussmittel 50 des zweiten Gehäuseteils 8 eine nach außen abgedichtete Druckkommunikation ausbildet.

In ähnlicher Weise ist die Kopplung zwischen dem Anschluss 13 für einen Mess- bzw. Spülkanal und dem zugehörigen ebenfalls beispielhaft konusförmig ausgebildeten Anschlussmittel 60 am ersten Gehäuseteil 4 ausgebildet. Wie aus Figur 3g ersichtlich ist, lässt sich in die Durchgangsöffnung 62 in dem zweiten Gehäuseteil 8 ein nicht dargestelltes Kopplungs- oder Tüllenteil einsetzen, welches dann den in Figur 1d dargestellten Anschluss 13 für den Mess- oder Spülkanal bildet. Dieses nicht dargestellte Kopplungs- oder Tüllenteil kann dann mit dem konusförmigen Anschlussmittel 60 druckdicht gekoppelt werden. Auf diese Weise kann über eine Leitung ein fluides Medium, insbesondere Luft oder eine Spülflüssigkeit, zur Wunde geleitet werden, um die Absaugung von Wundsekreten zu unterstützen. Typischerweise sind eine Mess- oder Spülleitung und die Saugleitung als wegwerfbare Einwegkomponenten Zubehörbestandteil zu dem zweiten Gehäuseteil; sie werden nach Gebrauch zusammen mit diesem entsorgt.

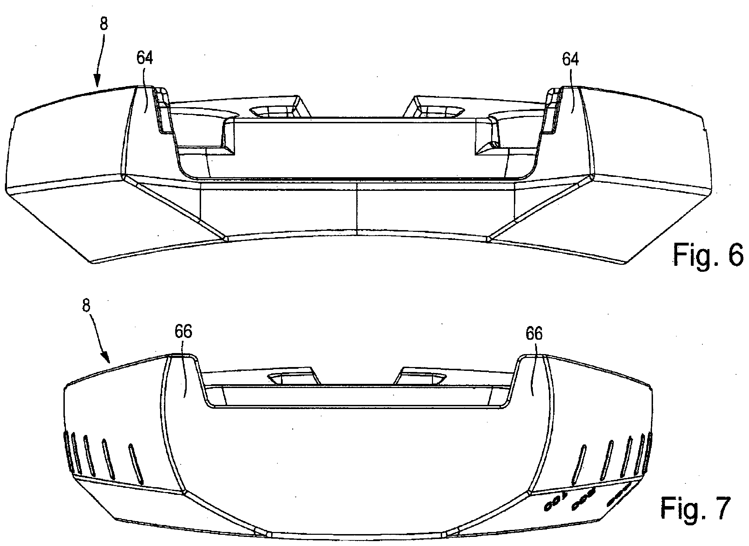

Schließlich verdeutlichen die Figuren 6 und 7 unterschiedlich große Standflächen 64 (mit ca. 7 cm²) und 66 (mit ca. 55 cm²) bei den verschieden großen zweiten Gehäuseteilen 8 der Ausführungsformen nach Figuren 1 und 4. Die Gehäuseteile 8 sind mit diesen Standflächen 64 bzw. 66 auf eine Unterlage abstellbar, wenn sie von dem ersten Gehäuseteil 4 abgenommen sind. Wenn sie am ersten Gehäuseteil 4 gehalten sind, so wird die Standfläche der Vorrichtung 2 von dem ersten Gehäuseteil 4 gebildet. Es können an den Standflächen der Gehäuseteile 4, 8 auch Fußelemente vorgesehen sein, wie dies aus den Figuren 1b, 2b, 4b ersichtlich ist.

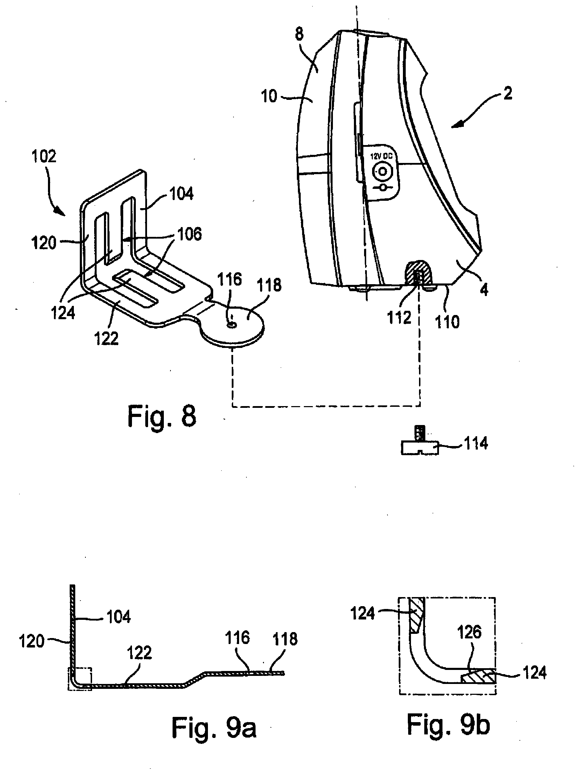

Figur 8 zeigt eine insgesamt mit dem Bezugszeichen 2 bezeichnete erfindungsgemäß ausgebildete Vorrichtung zur Bereitstellung von Unterdruck für medizinische Anwendungen und eine hiermit zusammenwirkende, jedoch im gelösten Zustand dargestellte Halteeinrichtung 102. Von der Halteeinrichtung 102 ist in dieser Figur 8 lediglich ein im Wesentlichen starres und beispielhaft als metallisches Biegestanzteil ausgebildetes Halteteil 104 dargestellt, welches eine Aufnahme 106 für ein in den nachfolgenden Figuren dargestelltes biegsames und vorzugsweise elastisch nachgiebiges bandförmiges Haltemittel 108 umfasst. Das starre Halteteil 104 ist zur lösbaren Befestigung an dem ersten Gehäuseteil 4 der Vorrichtung 2 ausgebildet. Hierfür ist an einer Unterseite 110 des ersten Gehäuseteils 4 eine Gewindeöffnung 112 ausgebildet, in die eine manuell und vorzugsweise werkzeuglos festziehbare und lösbare Klemmschraube 114 einschraubbar ist. Das starre Halteteil 104 weist hierfür eine Durchgangsöffnung 116 in einem vorzugsweise flächenhaft ausgebildeten Anlagebereich 118 auf.

Das Halteteil 104 ist aus einem metallischen Flachmaterialabschnitt auf die aus Figuren 9a und 9b ersichtliche winkelförmige Gestalt gebogen. Wird das Halteteil 104 mittels der Klemmschraube 114 mit der jeweiligen Ebene seiner Abschnitte senkrecht zur Zeichnungsebene in Figur 8 gegen das erste Gehäuseteil 4 der Vorrichtung 2 fixiert, so ist der zweite Gehäuseteil 8, der den Behälter 10 zur Aufnahme von Flüssigkeit bildet, zwischen dem einen Schenkel 120 des starren Halteteils 104 und dem ersten Gehäuseteil 4 der Halteeinrichtung 2 angeordnet. Der andere Schenkel 122 des starren Halteteils 104 unterfängt dabei den Behälter 8.

Man erkennt aus den Figuren 8 ff., dass die jeweilige an dem Halteteil 104 ausgebildete Aufnahme 106 als Schiebesitzanordnung ausgebildet ist. Sie umfasst hierfür jeweils einen fingerförmigen oder zungenförmigen frei auslaufenden Steg 124, der durch Ausstanzen aus dem metallischen Flachmaterialabschnitt gebildet ist; jedoch wären auch andere Gestaltungen denkbar.

Aus der Detaildarstellung nach Figur 9b erkennt man, dass der jeweilige Steg 124 an seinem freien Ende eine Abflachung oder Anfasung 126 aufweist, also verjüngt ausläuft. Hierdurch wird ein erleichtertes Aufschieben oder Aufstecken des noch zu beschreibenden bandförmigen Haltemittels 108 erreicht.

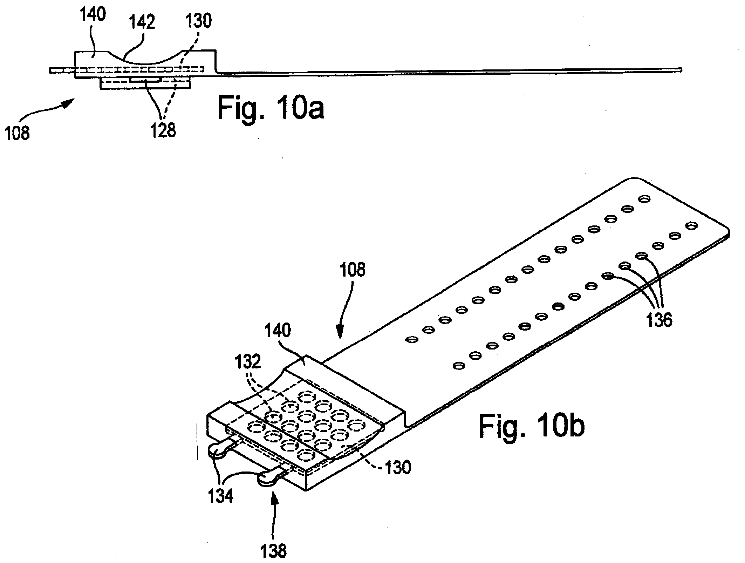

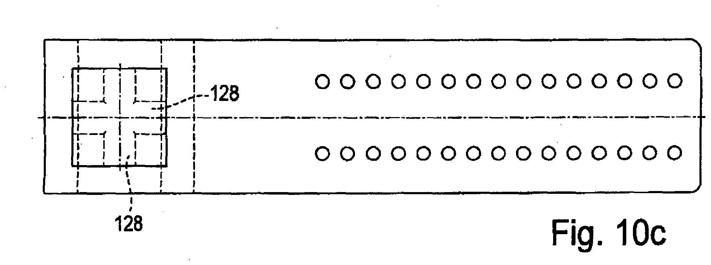

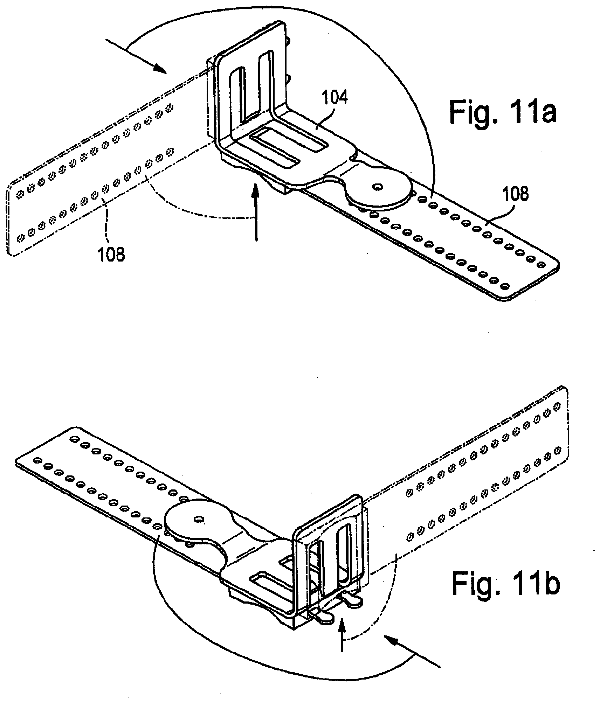

Die Figuren 10a und b zeigen in Seitenansicht und in perspektivischer Ansicht das biegsame bandförmige Haltemittel 108, welches aus einem insbesondere elastisch nachgiebigen Material, insbesondere auf Gummi-, Silikon-, Polymerbasis ausgebildet ist. Das bandförmige Haltemittel 108 umfasst zum Ankoppeln an das starre Halteteil 104 eine der Geometrie der zungenförmigen Stege 124 entsprechende kanalbildende Aussparung 128, mit der das bandförmige Haltemittel 108 auf den einen oder anderen Steg 124 aufschiebbar ist und somit die in Figur 11 a,b dargestellten verschiedenen Konfigurationen einnehmen kann. Wie noch besser aus Figur 10c ersichtlich ist, verlaufen zwei kanalbildende Aussparungen 128 rechtwinklig zueinander, so dass das bandförmige Haltemittel 108 in verschiedenen Ausrichtungen auf die fingerförmigen Stege 124 des starren Halteteils 104 aufschiebbar ist (s. Figuren 11a, b).

Bei dem bandförmigen Haltemittel 108 gemäß Figuren 10a, b ist zusätzlich zu dem biegsamen bandförmigen Material ein starres, vorzugsweise metallisches Verstärkungsteil 130 vorgesehen, welches Durchgangsöffnungen 132 aufweist und in das nachgiebige Material eingegossen oder eingespritzt ist. Das Verstärkungsteil 130 erstreckt sich mit beispielhaft zwei Vorsprüngen 134 aus dem nachgiebigen Material heraus. Diese Vorsprünge 134 bilden zusammen mit lochrasterartigen Öffnungen 136 in dem bandförmigen Haltemittel 108 mechanisch wirkende Verschlussmittel 138. Hierbei lässt sich der die Öffnungen 136 aufweisende Abschnitt des bandförmigen Haltemittels 108 um eine vertikale oder horizontale Strebe eines Patientenbetts oder Infusionsständers herumlegen und unter Ausübung von Zug werden zwei der Öffnungen 136 in Eingriff mit den Vorsprüngen 134 gebracht, so dass unter Zugspannung eine spielfreie Anordnung erreichbar ist. Zwar erweist sich die Gestaltung mit einem Verstärkungsteil 130 als vorteilhaft. Es wäre aber zumindest grundsätzlich denkbar, dass kein solches Verstärkungsteil vorhanden ist (wie in Figur 10c dargestellt). In diesem Fall könnten entsprechende Vorsprünge bei dem starren Halteteil 104 oder in anderer Weise bei dem bandförmigen Haltemittel 108 vorgesehen sein.

Man erkennt bei dem biegsamen bandförmigen Haltemittel 108 weiter einen verdickten Abschnitt 140, innerhalb dessen auch das erwähnte Verstärkungsteil 130 aufgenommen ist. Dieser verdickte Bereich 140 ist quer zur Bandebene dreidimensional konturiert ausgebildet. Er umfasst eine ungefähr konkav gewölbte Anlagezone 142, mit der die Halteeinrichtung dann gegen eine langgestreckte Strebe oder Stange angelegt werden kann. Dadurch, dass die Anlagezone 142 ebenfalls nachgiebig, vorzugsweise elastisch nachgiebig, ausgebildet ist, lässt sich eine große flächenhafte Anlage an die Strebe oder Stange erreichen.

In Figuren 11a und b sind zudem Pfeile dargestellt, welche die jeweilige Aufschieberichtung für das mit dem betreffenden Pfeil assoziierte bandförmige Haltemittel 108 bezeichnen. Jedes bandförmige Haltemittel 108 mit seinen zwei rechtwinklig zueinander orientierten kanalbildenden Aussparungen 128 lässt sich in vier Orientierungen an dem jeweiligen fingerförmigen Steg 124 des starren Halteteils 104 anordnen, so dass insgesamt acht verschiedene Anordnungen zweier bandförmiger Haltemittel 108 bezüglich des starren Halteteils 104 realisiert werden können.

Insgesamt gestattet die erfindungsgemäße Ausbildung der Vorrichtung 2 und der Halteeinrichtung 102 eine rasche und bedienerfreundliche Montierbarkeit der Vorrichtung an einem Patientenbett, einem Infusionsständer oder dergleichen.

## Patentansprüche

1. Vorrichtung (2) zur Bereitstellung von Unterdruck für medizinische Anwendungen, insbesondere zur Unterdruckbehandlung von Wunden am menschlichen oder tierischen Körper, mit einer Unterdruck erzeugenden Einrichtung und einem Behälter (10) zur Aufnahme von Körperflüssigkeiten, insbesondere von aus einer Wunde abgesaugten Wundsekreten, wobei der Behälter (10) lösbar an der Vorrichtung befestigbar ist und wobei ein Anschluss (12) für eine zum Körper führende Saugleitung vorgesehen ist, so dass eine Unterdruckkommunikation zwischen der Unterdruck erzeugenden Einrichtung, dem Behälter (10) und der zum Körper führenden Saugleitung herstellbar ist, und mit einer Halteeinrichtung (102) zum lösbaren Befestigen der Vorrichtung (2) an einer horizontalen oder vertikalen oder schräg geneigten Strebe oder Stange, **dadurch gekennzeichnet, dass** die Halteeinrichtung (102) ein an einem Gehäuse (4) der Vorrichtung befestigbares und hiervon lösbares im wesentlichen starres Halteteil (104) und ein biegsames, vorzugsweise elastisch nachgiebiges bandförmiges Haltemittel (108) umfasst, und dass das starre Halteteil (104) seinerseits eine Aufnahme (106) zur lösbaren Anordnung des biegsamen bandförmigen Haltemittels (108) aufweist, und dass das biegsame bandförmige Haltemittel (108) um eine horizontale oder vertikale oder schräg geneigte Strebe oder Stange herumlegbar und auf sich oder auf das Halteteil (104) zurückführbar und schließbar ist, derart dass unter Ausübung von Zugkraft auf das Haltemittel (108) und durch Schließen des Haltemittels (108) eine im wesentlichen spielfreie und dabei klemmschlüssige Fixierung der Vorrichtung (2) gegenüber der Strebe oder Stange erzielbar ist und wobei die an dem Halteteil ausgebildete Aufnahme (106) zur lösbaren Anordnung des biegsamen bandförmigen Haltemittels (108) eine Schiebesitzanordnung bildet, derart, dass das bandförmige Haltemittel (108) in einer linearen Richtung auf das starre Halteteil (104) aufschiebbar ist und die an dem Halteteil (104) ausgebildete Aufnahme (106) wenigstens einen fingerförmigen oder zungenförmigen Steg (124), insbesondere Flachsteg, aufweist, der beim Aufschieben des biegsamen bandförmigen Haltemittels (108) in eine kanalbildende Aussparung (128) des Haltemittels (108) eingreift, und bei der die an dem Halteteil (104) ausgebildete Aufnahme (106) so ausgebildet ist, dass das biegsame bandförmige Haltemittel (108) in zwei 90° zueinander einschließenden Orientierungen einer Bandlängsachse befestigbar ist und/oder das im wesentlichen starre Halteteil (104) wenigstens zwei Aufnahmen (106) zur lösbaren Anordnung je eines biegsamen bandförmigen Haltemittels (108) aufweist, wobei die Aufnahmen (106) so orientiert sind, dass die bandförmigen Haltemittel (108) in einem Winkel von vorzugsweise 90° zueinander und zu der Vorrichtung ausgerichtet sind sowie bei der das biegsame bandförmige Haltemittel (108) ein mechanisch wirkendes Verschlussmittel (138) aufweist, so dass es durch Zurückführen auf sich selbst oder auf das Halteteil (104) schließbar ist.

2. Vorrichtung nach Anspruch 1, **dadurch gekennzeichnet, dass** das im wesentlichen starre Halteteil (104) über eine Schraubenverbindung an dem Gehäuse (4) der Vorrichtung lösbar befestigbar ist, wobei hierfür eine Gewindeöffnung (112) in dem Gehäuse (4) der Vorrichtung ausgebildet ist oder ein Gewindebolzen vorgesehen sein kann.

3. Vorrichtung nach Anspruch 1 oder 2, **dadurch gekennzeichnet, dass** das im wesentlichen starre Halteteil (104) aus einem ebenen Flachmaterialabschnitt ausgebildet ist.

4. Vorrichtung nach einem oder mehreren der vorstehenden Ansprüche, **dadurch gekennzeichnet, dass** das im wesentlichen starre Halteteil (104) eine Winkelform aufweist.

5. Vorrichtung nach Anspruch 4, **dadurch gekennzeichnet, dass** an jedem Schenkel (120, 122) des winkelförmigen Halteteils (104) eine Aufnahme (106) zur lösbaren Anordnung je eines biegsamen, bandförmigen Haltemittels (108) ausgebildet ist.

6. Vorrichtung nach einem oder mehreren der vorstehenden Ansprüche, **dadurch gekennzeichnet, dass** das biegsame bandförmige Haltemittel (108) zwei vorzugsweise rechtwinklig zueinander orientierte kanalbildende Aussparungen (128) aufweist.

7. Vorrichtung nach nach einem oder mehreren der vorstehenden Ansprüche, **dadurch gekennzeichnet, dass** das mechanisch wirkende Verschlussmittel (138) von wenigstens einem Vorsprung (134) einerseits und von Durchgangsöffnungen (136) andererseits gebildet sind.

8. Vorrichtung nach Anspruch 7, **dadurch gekennzeichnet, dass** der wenigstens eine Vorsprung (134) in einer Ebene des biegsamen bandförmigen Haltemittels (108) oder in einer Ebene des starren Halteteils (104) erstreckt ist.

9. Vorrichtung nach einem oder mehreren der vorstehenden Ansprüche, **dadurch gekennzeichnet, dass** das biegsame bandförmige Haltemittel (108) ein starres, vorzugsweise metallisches, insbesondere als eingespritztes Einlegeteil ausgebildetes Verstärkungsteil (130) aufweist.

10. Vorrichtung nach Anspruch 9, **dadurch gekennzeichnet, dass** das Verstärkungsteil (130) den wenigstens einen Vorsprung (134) bildet.

11. Vorrichtung nach einem oder mehreren der vorstehenden Ansprüche, **dadurch gekennzeichnet, dass** das biegsame bandförmige Haltemittel (108) einen verdickten Bereich (140) aufweist, der quer zur Bandebene 3-dimensional konturiert ausgebildet ist, um eine nachgiebige Anlagezone (142) zur Anlage an eine Stange oder Strebe zu bilden.

12. Vorrichtung nach einem oder mehreren der vorstehenden Ansprüche, **dadurch gekennzeichnet, dass** das starre Halteteil (104) so ausgebildet ist, dass zwischen dessen Aufnahme (106) für das bandförmige Haltemittel (108) und dem Gehäuse (4) der Vorrichtung der lösbare Behälter (10) zur Aufnahme von Flüssigkeit vorgesehen ist.

13. Vorrichtung nach einem oder mehreren der vorstehenden Ansprüche, **dadurch gekennzeichnet**, die Halteeinrichtung (102) und ihr starres Halteteil (104) so ausgebildet und an dem Gehäuse (4) fixierbar sind, dass der Behälter (10) oder auch unterschiedlich dimensionierte Behälter (10), ohne die Halteeinrichtung (102) für die Vorrichtung (2) lösen zu müssen, von dem Gehäuse (4) abgenommen oder daran angesetzt werden können.

## Claims

1. Device (2) for generating a vacuum for medical applications, in particular for the vacuum therapy of wounds on a human or animal body, with a facility for generating a vacuum and a vessel (10) for receiving body fluids, in particular wound exudates suctioned out of a wound, wherein the vessel (10) can be detachably fastened to the device and wherein a connection (12) for a suction tube leading to the body is provided, so that vacuum communication between the facility for generating the vacuum, the vessel (10), and the suction tube leading to the body can be established, and with a holding facility (102) for detachably fastening of the device (2) on a horizontal or vertical or oblique strut or bar, **characterized in that** the holding facility (102) comprises an essentially rigid holding part (104), which can be attached to a housing (4) of the device and detached therefrom and comprises a flexible, preferably elastically compliant ribbon-shaped holding means (108), and that the rigid holding part (104) comprises a receptacle (106) for detachable disposition of the flexible ribbon-shaped holding means (108), and that the flexible ribbon-shaped holding means (108) can be placed around a horizontal or vertical or oblique strut or bar and can be turned back on itself or onto the holding part (104) and can be closed in such a way that, by exerting a tensile force on the holding means (108) and by closing the holding means (108), essentially clearance-free and clamped fastening of the device (2) to the strut or bar can be achieved and wherein the receptacle (106) constituted on the holding part for detachable disposition of the flexible ribbon-shaped holding means (108) constitutes a sliding-seat configuration in such a way that the ribbon-like holding means (108) can be pushed onto the rigid holding part (104) in a linear direction and the receptacle (106) constituted on the holding part (104) has at least one finger-shaped or tongue-shaped web (124), in particular a flat web, which engages in a duct-forming cutout (128) of the holding means (108) when the flexible ribbon-shaped holding means (108) is pushed on and **in that** the receptacle (106) constituted on the holding part (104) is constituted in such a way that the flexible ribbon-shaped holding means (108) can be fastened in two orientations of a longitudinal axis of the ribbon that extend at 90° to each other and/or the essentially rigid holding part (104) has at least two receptacles (106), each for the detachable disposition of one flexible ribbon-shaped holding means (108), wherein the receptacles (106) are oriented in such a way that the ribbon-like holding means (108) are aligned at an angle of preferably 90° with respect to each other and with respect to the device as well as the flexible ribbon-shaped holding means (108) has a mechanical-action closing means (138), such that it can be closed by turning back on itself or onto the holding part (104).

2. Device according to the claim 1, **characterized in that** the essentially rigid holding part (104) can be detachably fastened to the housing (4) of the device by means of a screw fastening, wherein, for this purpose, a threaded hole (112) is constituted in the housing (4) of the device or a threaded bolt can be provided.

3. Device according to the claim 1 or 2, **characterized in that** the essentially rigid holding part (104) is made of a flat, thin material section.

4. Device according to one or more of the previous claims, **characterized in that** the essentially rigid holding part (104) has an angular shape.

5. Device according to the claim 4, **characterized in that** a receptacle for detachable disposition of one flexible ribbon-shaped holding means (108) is constituted on each leg (120, 122) of the angle-shaped holding part (104).

6. Device according to one or more of the previous claims, **characterized in that** the flexible ribbon-shaped holding means (108) has a duct-forming cutout (128) or two preferably mutually perpendicularly oriented duct-forming cutouts (128).

7. Device according to one or more of the previous claims, **characterized in that** the mechanical-action closing means (138) is constituted by at least one projection (134) on one side and by through-holes (136) on the other side.

8. Device according to the claim 7, **characterized in that** the at least one projection (134) extends in a plane of the flexible ribbon-shaped holding means (108) or in a plane of the rigid holding part (104).

9. Device according to one or more of the previous claims, **characterized in that** the flexible ribbon-shaped holding means (108) has a reinforcing part (130) constituted as a rigid, preferably metal, in particular, injection molded part.

10. Device according to the claim 9, **characterized in that** the reinforcing part (130) defines the at least one projection (134).

11. Device according to one or more of the previous claims, **characterized in that** the flexible ribbon-shaped holding means (108) has a thicker region (140) that is constituted with 3-dimensional contouring, in the transverse direction with respect to ribbon plane, to form a compliant contact zone (142) for contacting a bar or strut.

12. Device according to one or more of the previous claims, **characterized in that** the rigid holding part (104) is constituted in such a way that the detachable vessel (10) for receiving liquids is provided between the receptacle (106) of the rigid holding part (104) for the ribbon-like holding means (108) and the housing (4) of the device.

13. Device according to one or more of the previous claims, **characterized in that** the holding facility (102) and its rigid holding part (104) are constituted in such a way and can be fastened to the housing (4) in such a way that the vessel (10), or also variously dimensioned vessels (10), can be removed from the housing (4) or mounted thereon without any need to detach the holding facility (102) for the device (2).

## Revendications

1. Dispositif (2) destiné à fournir une pression négative pour des applications médicales, en particulier pour le traitement par pression négative de plaies sur le corps humain ou animal, comprenant un dispositif générant une pression négative et un récipient (10) de réception de liquides corporels, en particulier d'exsudats de plaie aspirés d'une plaie, ledit récipient (10) pouvant être fixé de manière amovible sur le dispositif et un raccord (12) étant prévu pour une conduite d'aspiration menant vers le corps, de sorte que l'on peut établir une communication à pression négative entre ledit dispositif générant une pression négative, ledit récipient (10) et ladite conduite d'aspiration menant vers le corps, et comprenant un dispositif de maintien (102) pour fixer d'une manière amovible le dispositif (2) sur une entretoise ou tige horizontale ou verticale ou inclinée obliquement, **caractérisé par le fait que** ledit dispositif de maintien (102) comprend une partie de maintien (104) pour l'essentiel rigide apte à être fixée sur un boîtier (4) du dispositif et à être détachée de celui-ci ainsi qu'un moyen de maintien (108) en forme de bande qui est souple, de préférence élastiquement flexible, et que ladite partie de maintien (104) rigide présente, de son côté, un logement (106) destiné à disposer d'une manière amovible ledit moyen de maintien (108) souple en forme de bande et que ledit moyen de maintien (108) souple en forme de bande peut être placé autour d'une entretoise ou tige horizontale ou verticale ou inclinée obliquement et peut être ramené sur lui-même ou sur ladite partie de maintien (104) et être fermé, de telle sorte que, en exerçant une force de traction sur ledit moyen de maintien (108) et en fermant ledit moyen de maintien (108), on peut obtenir une fixation du dispositif (2) par rapport à ladite entretoise ou tige, qui est pour l'essentiel exempte de jeu tout en étant entraînée par serrage et dans lequel le logement (106) réalisé sur la partie de maintien et destiné à disposer de manière amovible ledit moyen de maintien (108) souple en forme de bande forme une disposition à logement coulissant de telle sorte que le moyen de maintien (108) en forme de bande peut être placé par coulissement dans une direction linéaire sur ladite partie de maintien (104) rigide et ledit logement (106) réalisé sur la partie de maintien (104) présente au moins une traverse (124) en forme de doigt ou en forme de languette, en particulier traverse plate, qui, lorsque ledit moyen de maintien (108) souple en forme de bande est placé par coulissement, s'engage dans un évidement (128) formant canal du moyen de maintien (108) et ledit logement (106) réalisé sur la partie de maintien (104) est réalisé de telle manière que le moyen de maintien (108) souple en forme de bande peut être fixé dans deux orientations d'un axe longitudinal de bande qui font 90° l'une par rapport à l'autre et/ou ladite partie de maintien (104) pour l'essentiel rigide présente au moins deux logements (106) destinés à disposer de manière amovible respectivement un moyen de maintien (108) souple en forme de bande, lesdits logements (106) étant orientés de manière à ce que les moyens de maintien (108) en forme de bande soient orientés l'un par rapport à l'autre et audit dispositif à un angle de 90° de préférence aussi bien que ledit moyen de maintien (108) souple en forme de bande présente un moyen de fermeture (138) agissant mécaniquement de sorte qu'il peut être fermé en étant ramené sur lui-même ou sur ladite partie de maintien (104).

2. Dispositif selon la revendication 1, **caractérisé par le fait que** ladite partie de maintien (104) pour l'essentiel rigide peut être fixée de manière amovible par une liaison vissée sur ledit boîtier (4) du dispositif; à cette fin, une ouverture taraudée (112) étant ménagée dans le boîtier (4) du dispositif ou un boulon fileté pouvant être prévu.

3. Dispositif selon la revendication 1 ou 2, **caractérisé par le fait que** ladite partie de maintien (104) pour l'essentiel rigide est réalisée à partir d'une portion plane en matériau plat.

4. Dispositif selon l'une ou plusieurs des revendications précédentes, **caractérisé par le fait que** ladite partie de maintien (104) pour l'essentiel rigide présente une forme angulaire.

5. Dispositif selon la revendication 4, **caractérisé par le fait que** sur chaque branche (120, 122) de la partie de maintien (104) angulaire est réalisé un logement (106) destiné à disposer de manière amovible respectivement un moyen de maintien (108) souple en forme de bande.

6. Dispositif selon l'une ou plusieurs des revendications précédentes, **caractérisé par le fait que** ledit moyen de maintien (108) souple en forme de bande présente un évidement (128) formant canal ou deux évidements (128) formant canal orientés de préférence à angle droit l'un par rapport à l'autre.

7. Dispositif selon l'une ou plusieurs des revendications précédentes, **caractérisé par le fait que** ledit moyen de fermeture (138) agissant mécaniquement est formé d'au moins une projection (134) d'une part et d'ouvertures de passage (136) d'autre part.

8. Dispositif selon la revendication 7, **caractérisé par le fait que** ladite au moins une projection (134) s'étend dans un plan du moyen de maintien (108) souple en forme de bande ou dans un plan de la partie de maintien (104) rigide.

9. Dispositif selon l'une ou plusieurs des revendications précédentes, **caractérisé par le fait que** ledit moyen de maintien (108) souple en forme de bande présente une pièce de renfort (130) rigide, de préférence métallique, réalisée en particulier en tant que pièce d'insertion injectée.

10. Dispositif selon la revendication 9, **caractérisé par le fait que** ladite pièce de renfort (130) forme ladite au moins une projection (134).

11. Dispositif selon l'une ou plusieurs des revendications précédentes, **caractérisé par le fait que** ledit moyen de maintien (108) souple en forme de bande présente une zone épaissie (140) qui, transversalement au plan de la bande, est réalisée de manière à être contourée en trois dimensions afin de former une zone d'appui (142) flexible pour l'appui sur une tige ou entretoise.

12. Dispositif selon l'une ou plusieurs des revendications précédentes, **caractérisé par le fait que** ladite partie de maintien (104) rigide est réalisée de telle manière que ledit récipient (10) amovible de réception de liquide est prévu entre le logement (106) de celle-ci pour le moyen de maintien (108) en forme de bande et ledit boîtier (4) du dispositif.

13. Dispositif selon l'une ou plusieurs des revendications précédentes, **caractérisé par le fait que** ledit dispositif de maintien (102) et sa partie de maintien (104) rigide sont réalisés et peuvent être fixés sur le boîtier (4) de telle manière que ledit récipient (10) ou aussi des récipients (10) à dimensions différentes peut/peuvent être retiré(s) du boîtier (4) ou être positionné(s) sur celui-ci sans devoir détacher le dispositif de maintien (102) pour le dispositif (2).
